# EUROPEAN PATENT APPLICATION

(11) **EP 4 670 516 A1**
(43) Date of publication of application: **31.12.2025**
(21) Application number: 24759836.0
(22) Date of filing: 14.02.2024
(51) Int. Cl.: A23P 20/10, A61K 9/28, A23P 20/18, A23L 29/262, A23L 29/10

(54) **COMPOSITION FOR FILM COATING, AND FILM COATING LIQUID COMPRISING SAME**

(30) Priority: 20.02.2023 KR 20230022493; 25.05.2023 KR 20230067967
(71) Applicant: Dong-A Pharmaceutical Co., Ltd., Seoul 02587 (KR)
(72) Inventor: KIM, Soyeon, Yongin-si, Gyeonggi-do 17073 (KR); PARK, Junwoo, Yongin-si, Gyeonggi-do 17073 (KR); KIM, Sungrae, Yongin-si, Gyeonggi-do 17073 (KR)
(74) Representative: Gille Hrabal Partnerschaftsgesellschaft mbB Patentanwälte
(86) International application number: PCT/IB2024/051365
(87) International publication number: WO 2024/176050

(57) **Abstract**

The present invention relates to a film coating composition, and specifically to: a film coating composition having excellent safety and shielding properties; and a film coating liquid comprising same.

## Description

### Technical Field

The present invention relates to a film coating composition and a film coating liquid including the same, and specifically to a film coating composition having uniform coatability without a color difference, excellent safety and high opacity, and a film coating liquid including the same.

### Background Art

Titanium dioxide, which is a compound with a high refractive index, may provide excellent whiteness and opacity even in small amounts compared to other raw materials, and may block wavelengths in the ultraviolet range depending on particle sizes, and thus is known to be suitable for use in protecting coated products. Accordingly, titanium dioxide has been used as a pigment or opacifying agent in paints or coating materials, and has also been widely used in various fields of food, pharmaceuticals and the like, such as cosmetics, toothpaste, confectionery, bakery, gum, preparations and the like.

However, as the carcinogenicity issue of titanium dioxide has recently arisen and titanium dioxide has been classified as a carcinogenic substance, the use of titanium dioxide as a food additive has been prohibited in some countries including Europe, thus urging to develop a substance which may replace titanium dioxide.

As a substitute for titanium dioxide, calcium phosphate, isomalt, etc. have problems such as low whiteness, opacity, and opacifying efficiency compared to titanium dioxide, thus resulting in a dark final product or an exposed surface of a material to be coated. In addition, when increasing the amount of coating to increase the opacity, there are problems such as uneven color difference, rough surface texture and the like. In particular, main ingredients of health functional foods, etc., vitamins for nutritional supplement, minerals, etc., which are used after being extracted and refined from food, have their own colors, so when these materials are coated, there are disadvantages in that sufficient opacity is not secured and the final product has an untidy appearance.

Thus, there is a need to develop a film coating composition, which is not only safe without containing titanium dioxide, but also has high opacity capable of effectively covering both materials, which are not difficult to make opaque on the surface because of containing a large amount of commonly used white excipients, and materials, which are not easy to make opaque on the surface because of containing stains or dark colors.

### Disclosure

### Technical Problem

In order to solve the problems of the related art, the present invention may provide a film coating composition which is safe without containing titanium dioxide, and in which a coating layer formed using the film coating composition has high opacity, stability, and surface appearance. In addition, the present invention may provide a film coating liquid including the film coating composition, and a formulation such as a tablet, etc., or a food having a coating layer coated using the film coating composition.

### Solution to Problem

Calcium carbonate (CaCO₃) has an excellent whiteness index, but has a lower refractive index than that of titanium dioxide, and thus has a disadvantage in that opacity is deteriorated. In order to solve the problem of such a low opacity, a method of increasing the amount of film coating per se may be generally used, and the amount of film coating may be increased from 1.5 times to 3 times or more depending on the color or the degree of stains on the surface of the materials to be coated. However, such a method is not only uneconomical, but also inefficient because the method overshadows excellent film strength, identification of imprints, and the like, which are advantages of film coating, and lengthens a processing time. In addition, preparations, food or the like may be exposed to the outside such as air, etc., for a long time during the process, thereby causing quality deterioration, safety, and stability problems.

Moreover, a method of improving opacity by increasing the content of calcium carbonate in the coating composition may be considered. However, in order to increase the content of calcium carbonate, the proportion of other components such as a polymer, etc., in the composition may be inevitably reduced, which causes difficulties in forming a coating layer. Furthermore, since calcium carbonate has a feature of precipitating due to its density, an excessive amount of calcium carbonate may cause layer separation of the film coating liquid.

As a result of intensive efforts to solve the above problems, the present inventors have confirmed that the above objects are achieved by using the film coating composition of the present invention including calcium carbonate and hydroxypropyl methylcellulose (HPMC), thereby completing the present invention.

The present invention provides a film coating composition including:
60 wt% to 85 wt% of calcium carbonate (CaCO₃) and 10 wt% to 38 wt% of hydroxypropyl methylcellulose compared to the total weight of the composition.

The film coating composition may be in a solid phase, but is not limited thereto, and may be specifically in a powder form.

According to one exemplary embodiment of the present invention, the film coating composition may include 60 wt% to 85 wt% of calcium carbonate (CaCO₃), 10 wt% to 38 wt% of hydroxypropyl methylcellulose and 0.5 wt% to 3 wt% of a plasticizer compared to the total weight of the composition, may optionally further include an additive, and does not include titanium dioxide.

In this case, the additive may be included in a remaining amount such that the total amount of the film coating composition is 100 wt%.

The hydroxypropyl methylcellulose (HPMC) may be a mixture of low-viscosity HPMC and high-viscosity HPMC, and specifically may be a mixture of HPMC having a viscosity of about 100 cps or less and HPMC having a viscosity of about 3000 cps or more in a weight ratio of about 1:1 to about 15:1.

More specifically, the hydroxypropyl methylcellulose may be a mixture of HPMC having a viscosity of about 6 to about 100 cps and HPMC having a viscosity of about 3000 to about 12000 cps in a weight ratio of about 1:1 to about 15:1, more specifically a mixture of HPMC having a viscosity of about 15 to about 100 cps and HPMC having a viscosity of about 3000 to about 10000 cps in a weight ratio of about 1:1 to about 15:1, and much more specifically a mixture of HPMC having a viscosity of about 15 to about 50 cps and HPMC having a viscosity of about 3000 to about 5000 cps in a weight ratio of about 1:1 to about 15:1.

For example, the present invention provides a film coating composition, in which the HPMC having a viscosity of about 100 cps or less may be HPMC 2910, and the HPMC having a viscosity of about 3000 cps or more may be HPMC 2208.

According to an exemplary embodiment of the present invention, a composition including calcium carbonate (CaCO₃), hydroxypropyl methylcellulose, and a plasticizer in the content range and not including titanium dioxide may exhibit a whiteness index and high opacity.

The film coating composition of the present invention does not include titanium dioxide.

The film coating composition of the present invention may be a coating composition which does not contain titanium dioxide, and thus may be safe and provide a whiteness index and high opacity. By using the film coating composition of the present invention, a film coating liquid having an appropriate viscosity may be prepared, and a final product having a uniform color may be prepared by effectively and uniformly shielding the surface of the material to be coated. In addition, it may be unnecessary to increase the amount of coating for improving opacity, and it may be possible to manufacture a final product having a solid surface due to excellent adhesion to the material to be coated, and having excellent discrimination of imprints in the material to be coated as well as clearness. Moreover, a product prepared through a production process with cost and time efficiency as an effect accompanying high opacity may be provided.

### Calcium carbonate (CaCO₃)

The calcium carbonate may act as a whitening agent and/or an opacifying agent in a film coating composition, and may provide film coating with properties suitable as a coating material through the high whiteness and hydrophilicity of calcium carbonate.

The calcium carbonate may be heavy calcium carbonate obtained by physically pulverizing limestone, or precipitated calcium carbonate prepared as fine particles, which are obtained by chemical recrystallization through calcination, extinguishing reaction, etc. More specifically, it may be precipitated calcium carbonate, and precipitated calcium carbonate may be preferable to maintain the quality of the film coating constant due to its uniform particle size and particle shape.

The calcium carbonate may be included in an amount of about 60 wt% to about 85 wt%, specifically about 65 wt% to about 80 wt%, or about 70 wt% to about 85 wt%, or about 70 wt% to about 80 wt%, and more specifically about 75 wt% to about 80 wt% compared to the total weight of the film coating composition. When the content of calcium carbonate is within the above-mentioned range, the coating layer having excellent opacity may be firmly formed without increasing the amount of coating.

### Hydroxypropyl methylcellulose (HPMC)

The hydroxypropyl methylcellulose (HPMC) may be included in the film coating composition to serve as a polymer for forming a coating film, and may serve to form a continuous film when the solvent in the coating solution is evaporated. In addition, when HPMC is used, a solid coating layer may be formed by securing sufficient viscosity.

In one exemplary embodiment of the present invention, the hydroxypropyl methylcellulose (HPMC) may be a mixture of low-viscosity HPMC and high-viscosity HPMC, and the low-viscosity HPMC and the high-viscosity HPMC may have a weight ratio of about 1:1 to about 15:1, and more specifically about 1:1 to about 10:1.

In one exemplary embodiment of the present invention, the viscosity (cps) may refer to an average viscosity in an aqueous solution of 2%w/w at 20°C, and may be measured according to the measurement criteria of each HPMC manufacturer.

In one exemplary embodiment of the present invention, the low-viscosity HPMC may have a viscosity of about 100 cps or less, specifically about 6 to about 100 cps, more specifically about 15 to about 100 cps, and more specifically about 15 to about 50 cps.

In one exemplary embodiment of the present invention, the high-viscosity HPMC may have a viscosity of about 3000 cps or more, specifically about 3000 to about 12000 cps, more specifically about 3000 to about 10000 cps, and more specifically about 3000 to about 5000 cps.

In one exemplary embodiment of the present invention, the hydroxypropyl methylcellulose (HPMC) may be a mixture of HPMC having a viscosity of about 100 cps or less and HPMC having a viscosity of about 3000 cps or more.

In exemplary embodiments of the present invention, when the low-viscosity HPMC is used, the ability of forming the film coating layer may be improved.

In one exemplary embodiment of the present invention, when the high-viscosity HPMC is used, a gelation phenomenon or a phenomenon of excessively increased viscosity, which may occur during a process, may be prevented to prevent a coating defect. In addition, it may be possible to prevent a phenomenon in which the raw material is not well dissolved in the solvent.

As one example of the present invention, the hydroxypropyl methylcellulose may be a mixture of low-viscosity HPMC and high-viscosity HPMC, for example, a mixture of HPMC having a viscosity of about 100 cps or less and HPMC having a viscosity of about 3000 cps or more, specifically a mixture of HPMC having a viscosity of about 6 to about 100 cps and HPMC having a viscosity of about 3000 to about 12000 cps, more specifically a mixture of HPMC having a viscosity of about 15 to about 100 cps and HPMC having a viscosity of about 3000 to about 10000 cps, and more specifically a mixture of HPMC having a viscosity of about 15 to about 50 cps and HPMC having a viscosity of about 3000 to about 5000 cps.

In exemplary embodiments of the present invention, the HPMC may include the use of low-viscosity HPMC and high-viscosity HMPC in a weight ratio of about 1:1 to about 15:1, specifically about 1:1 to about 10:1, and more specifically 1:1, 2:1, 2.25:1, 3:1, 4:1, 5:1, 6:1, 24:3.5, 7:1, 8:1, 9:1, 34.5:3.5 or 10:1.

In exemplary embodiments of the present invention, the HPMC may include the use of HPMC having a viscosity of about 100 cps or less and HPMC having a viscosity of about 3000 cps or more in a weight ratio of about 1:1 to about 15:1, specifically about 1:1 to about 10:1, and more specifically 1:1, 2:1, 2.25:1, 3:1, 4:1, 5:1, 6:1, 24:3.5, 7:1, 8:1, 9:1, 34.5:3.5 or 10:1.

In exemplary embodiments of the present invention, the HPMC may be a mixture of HPMC having a viscosity of about 6 to about 100 cps and HPMC having a viscosity of about 3000 to 12000 cps in a weight ratio of about 1:1 to about 15:1, specifically about 1:1 to about 10:1, and more specifically 1:1, 2:1, 2.25:1, 3:1, 4:1, 5:1, 6:1, 24:3.5, 7:1, 8:1, 9:1, 34.5:3.5 or 10:1.

In exemplary embodiments of the present invention, the HPMC may be a mixture of HPMC having a viscosity of about 15 to about 100 cps and HPMC having a viscosity of about 3000 to 10000 cps in a weight ratio of about 1:1 to about 15:1, specifically about 1:1 to about 10:1, and more specifically 1:1, 2:1, 2.25:1, 3:1, 4:1, 5:1, 6:1, 24:3.5, 7:1, 8:1, 9:1, 34.5:3.5 or 10:1.

In exemplary embodiments of the present invention, the HPMC may be a mixture of HPMC having a viscosity of about 15 to about 50 cps and HPMC having a viscosity of about 3000 to about 5000 cps in a weight ratio of about 1:1 to about 15:1, and specifically about 1:1 to about 10:1. More specifically, the HPMC may include the use of HPMC having a viscosity of about 15 to about 50 cps and HPMC having a viscosity of about 3000 to 5000 cps in a weight ratio of 1:1, 2:1, 2.25:1, 3:1, 4:1, 5:1, 6:1, 7:1, 8:1, 9:1 or 10:1.

When the weight ratio is within the above-mentioned range, an appropriate viscosity may be provided to the coating liquid.

The HPMC used herein may include at least one selected from HPMC 2910, HPMC 2208, and HPMC 2206, but is not limited thereto.

As one example of the present invention, the HPMC having a viscosity of about 100 cps or less used herein may include HPMC 2910.

As one example of the present invention, the HPMC having a viscosity of about 3000 cps or more used herein may include HPMC 2208.

The HPMC 2910 and HPMC 2208 may be classified according to the degree of substitution between a methoxy group and a hydroxypropoxy group, and the HPMC 2910 may have a methoxy group substitution degree of about 28 wt% to about 30 wt% and a hydroxypropoxy group substitution degree of about 7 wt% to about 12 wt%.

Compared to HPMC 2910, HPMC 2208 may have a feature of relatively higher hydrophilicity due to a smaller methoxy group ratio. Like other polymers, the higher the ratio of molecules having a large molecular weight (high degree of polymerization), the higher the viscosity. The HPMC 2208 may have a methoxy group substitution degree of about 27 wt% to about 30 wt% and a hydroxypropoxy group substitution degree of about 4 wt% to about 7.5 wt%.

When HPMC 2910 and HPMC 2208 are used together as hydroxypropyl methylcellulose, it may be possible to provide an advantageous effect in securing hydrophilic properties suitable for the purpose of use.

In the present specification, the viscosity (cps) of HPMC may refer to an average viscosity in an aqueous solution of 2%w/w at 20°C, and may be a value measured according to the measurement criteria of each HPMC manufacturer.

In exemplary embodiments of the present invention, HPMC having a viscosity of about 100 cps or less may be one in which the viscosity is included in a range of about 3 to about 100 cps when measured in an aqueous solution of 2%w/w at 20°C, and specifically the viscosity is 3 cps, 4.5 cps, 5 cps, 6 cps, 15 cps, or 50 cps. For example, HPMC 2906, HPMC 2910, and the like may be included, which may more specifically include products under the name of Metollose 60SH-50 (manufactured by Shin-Etsu Chemical Co., Ltd.) (viscosity of 2% aqueous solution at 20°C: about 50 cps), pharmacoat 603 (manufactured by Shin-Etsu Chemical Co., Ltd.) (viscosity of 2% aqueous solution at 20°C: about 3 cps), pharmacoat 645 (manufactured by Shin-Etsu Chemical Co., Ltd.) (viscosity of 2% aqueous solution at 20°C: about 4.5 cps), pharmacoat 606 (manufactured by Shin-Etsu Chemical Co., Ltd.) (viscosity of 2% aqueous solution at 20°C: about 6 cps), pharmacoat 615 (manufactured by Shin-Etsu Chemical Co., Ltd.) (viscosity of 2% aqueous solution at 20°C: about 15 cps), etc.

In exemplary embodiments of the present invention, HPMC having a viscosity of about 3000 cps or more may be one in which the viscosity is included in a range of about 3000 to about 10000 cps when measured in an aqueous solution of 2%w/w at 20°C, and specifically the viscosity is about 4000 cps. For example, HPMC 2208, and the like may be included, which may more specifically include products under the name of Metollose 90SH-4000 (manufactured by Shin-Etsu Chemical Co., Ltd.) (viscosity of 2% aqueous solution at 20°C: about 4000 cps), etc.

The hydroxypropyl methylcellulose may be included in an amount of about 10 wt% to about 38 wt% compared to the total weight of the film coating composition. When the content of hydroxypropyl methylcellulose is within the above-mentioned range, formation of the coating layer may be advantageous.

### Plasticizer

The film coating composition of the present invention may further include a plasticizer. The plasticizer may impart flexibility to a film coating layer, and thus may help the formation of a film to facilitate the formation of the film coating layer.

Specific examples of the plasticizer may include at least one selected from di-sorbitol, di-mannitol, glycerin, polyethylene glycol, triacetin, acetyltriethyl citrate, and triethyl citrate, but are not limited thereto.

The plasticizer may be included in an amount of about 0.5 wt% to about 3 wt%, and more specifically about 1 wt% to about 3 wt% compared to the total weight of the film coating composition. When the content of the plasticizer is within the above-mentioned range, it may be possible to provide an effect of increasing the durability of the coating film.

### Other additives

The film coating composition of the present invention may further include one or more additive selected from a surfactant, an antioxidant, a sweetener, a flavoring agent, a glazing agent, and a colorant, but is not limited thereto. The additive may be one which is pharmaceutically acceptable.

The surfactant may weaken the surface hydrophobicity imparted by the lubricant component essentially used in the compression process of tablets, thereby promoting the adhesion of the film coating liquid to the tablet surface. Specific examples of the surfactant may include sodium lauryl sulfate (SLS), Tween, Span, and the like, but are not limited thereto.

The antioxidant may improve the stability of the film coating composition and may prevent oxidation and discoloration of the coating layer. Specific examples of the antioxidant may include tert-butyl hydroquinone (TBHQ), erythorbic acid, and the like, but are not limited thereto.

The sweetener may be used to remove unpleasant tastes and impart a desired taste. Specific examples of the sweetener may include sugar, sucralose, enzyme-treated stevia, erythritol, xylitol, and the like, but are not limited thereto.

The flavoring agent may be used to remove unpleasant scents and impart a desired scent, and a conventional one may be used in the art.

The glazing agent may be used to impart gloss and a soft touch to a final coated product. Specific examples of the glazing agent may include carnauba wax, candelilla wax, rice bran wax, and the like, but are not limited thereto.

The colorant may be used to change the color of the final coated product to facilitate identification of the product or to improve preference. Specific examples of the colorant may include riboflavin, beta-carotene, monascus pigment, safflower pigment, and the like, but are not limited thereto.

The additive may be included in an amount of about 0.1 wt% to about 5 wt% compared to the total weight of the film coating composition, and is not particularly limited as long as it is conventionally used in the art.

### Film coating liquid

The present invention provides a film coating liquid including the film coating composition.

The present invention provides a film coating liquid including the film coating composition and a solvent.

The present invention provides a film coating liquid including about 5 wt% to about 25 wt%, specifically about 10 wt% to about 20 wt% of the film coating composition compared to the total weight of the film coating liquid. In this case, a remaining amount thereof may be a solvent.

In other words, the present invention provides a film coating liquid including about 5 wt% to about 25 wt%, specifically about 10 wt% to about 20 wt%, and more specifically about 10 wt% to about 15 wt% of the film coating composition and the remaining amount of the solvent compared to the total weight of the film coating liquid. As one example, the film coating composition may be included in an amount of about 10 wt%, about 11 wt%, about 12 wt%, about 13 wt%, about 14 wt%, about 15 wt%, about 16 wt%, about 17 wt%, about 18 wt%, about 19 wt%, or about 20 wt% compared to the total weight of the film coating liquid.

When the content of the film coating composition in the film coating liquid is within the above-mentioned range, there may be an effect of reducing a manufacturing process failure rate, and the film coating layer may be stably and firmly formed.

The solvent is not particularly limited as long as it is capable of dispersing or dissolving the film coating composition, and more preferably may be one which is capable of dissolving the film coating composition. Examples of the solvent may include at least one selected from water and ethanol. More preferably, the solvent may be water, specifically purified water.

In exemplary embodiments of the present invention, the film coating liquid may have a viscosity of about 100 to about 500 cps at 25°C. When the viscosity of the film coating liquid is within the above-mentioned range, it may be advantageous to apply the same to a coating process.

In exemplary embodiments of the present invention, each component, and the content and physical properties of each component included in the film coating composition included in the film coating liquid may be as described above.

The film coating liquid may be prepared by dispersing or dissolving the film coating composition of the present invention in a solvent, and may be for spray coating a formulation or food.

The formulation may be a solid formulation, and specifically may be at least one selected from a tablet, a granule, and a pellet, and more specifically may be the tablet, but is not limited thereto.

The food is not particularly limited as long as it has a shape capable of coating the coating liquid of the present invention, and may be a solid-phase food.

The food or formulation may be uncoated or already coated.

The present invention provides a formulation or food coated using the film coating composition.

In addition, the present invention provides a formulation or food coated using a film coating liquid including the film coating composition. In other words, the present invention provides a formulation or food, such as a tablet, etc., coated with a film coating liquid including the film coating composition and a solvent.

Matters mentioned in each of the items of the present invention, that is, the film coating composition, the film coating liquid and the coated formulation or food may be applied the same, if not contradictory to each other. Besides, the terms and abbreviations used in the present specification may have their original meanings, unless defined otherwise.
(1) A film coating composition according to the present invention may include:
   60 wt% to 85 wt% of calcium carbonate (CaCO₃); and 10 wt% to 38 wt% of hydroxypropyl methylcellulose (HPMC) compared to the total weight of the composition.
(2) With regard to the film coating composition according to above (1), it may be that the hydroxypropyl methylcellulose is a mixture of HPMC having a viscosity of 100 cps or less and HPMC having a viscosity of 3000 cps or more.
**(3)** With regard to the film coating composition according to above **(1)** or **(2),** it may be that the hydroxypropyl methylcellulose is a mixture of HPMC having a viscosity of 6 to 100 cps and HPMC having a viscosity of 3000 to 12000 cps.
**(4)** With regard to the film coating composition according to any one of above **(1)** to **(3),** it may be that the hydroxypropyl methylcellulose is a mixture of HPMC having a viscosity of 15 to 100 cps and HPMC having a viscosity of 3000 to 10000 cps.
**(5)** With regard to the film coating composition according to any one of above **(1)** to **(4),** it may be that the hydroxypropyl methylcellulose is a mixture of HPMC having a viscosity of 15 to 50 cps and HPMC having a viscosity of 3000 to 5000 cps.
**(6)** With regard to the film coating composition according to any one of above **(1)** to **(5)**, it may be that the hydroxypropyl methylcellulose is a mixture of HPMC having a viscosity of 100 cps or less and HPMC having a viscosity of 3000 cps or more, and the HPMC having a viscosity of 100 cps or less and the HPMC having a viscosity of 3000 cps or more have a weight ratio of 1:1 to 15:1.
**(7)** With regard to the film coating composition according to any one of above **(1)** to **(6),** it may be that the hydroxypropyl methylcellulose is a mixture of HPMC having a viscosity of 100 cps or less and HPMC having a viscosity of 3000 cps or more, and the HPMC having a viscosity of 100 cps or less and the HPMC having a viscosity of 3000 cps or more have a weight ratio of 1:1 to 10:1.
**(8)** With regard to the film coating composition according to any one of above **(1)** to **(7),** it may be that the hydroxypropyl methylcellulose is a mixture of HPMC having a viscosity of 100 cps or less and HPMC having a viscosity of 3000 cps or more, and the HPMC having a viscosity of 100 cps or less is HPMC 2910.
**(9)** With regard to the film coating composition according to any one of above **(1)** to **(8),** it may be that the hydroxypropyl methylcellulose is a mixture of HPMC having a viscosity of 100 cps or less and HPMC having a viscosity of 3000 cps or more, and the HPMC having a viscosity of 3000 cps or more is HPMC 2208.
**(10)** With regard to the film coating composition according to any one of above **(1)** to **(9),** it may be that the composition further includes a plasticizer.
**(11)** With regard to the film coating composition according to any one of above **(1)** to **(10),** it may be that the composition further includes a plasticizer, and the plasticizer is included in an amount of 0.5 wt% to 3 wt% compared to the total weight of the composition.
**(12)** With regard to the film coating composition according to any one of above **(1)** to **(11),** it may be that the composition further includes a plasticizer, and the plasticizer is at least one selected from di-sorbitol, di-mannitol, glycerin, polyethylene glycol, triacetin, acetyltriethyl citrate and triethyl citrate.
**(13)** With regard to the film coating composition according to any one of above **(1)** to **(12),** it may be that the composition is for coating food or formulation.
**(14)** With regard to the film coating composition according to any one of above **(1)** to **(13),** it may be that the composition is for spray coating.
**(15)** With regard to the film coating composition according to any one of above **(1)** to **(14),** it may be that the composition includes calcium carbonate in an amount of about 65 wt% to about 80 wt%, or about 70 wt% to about 85 wt%, or about 70 wt% to about 80 wt%, or about 75 wt% to about 80 wt%.
**(16)** A film coating liquid according to the present invention may include:
   a film coating composition according to any one of above **(1)** to **(15);** and a solvent.
**(17)** With regard to the film coating liquid according to above **(16),** it may be that the film coating liquid includes 5 wt% to 25 wt% of the film coating composition according to above **(1)** to **(15)** compared to the total weight of the film coating liquid.
**(18)** With regard to the film coating liquid according to above **(16)** or **(17),** it may be that the film coating liquid includes the film coating composition according to any one of above **(1)** to **(15);** and a solvent, and the solvent is water or ethanol.
**(19)** With regard to the film coating liquid according to any one of above **(16)** to **(18),** it may be that the film coating liquid is for coating food or formulation.
**(20)** With regard to the film coating liquid according to any one of above **(16)** to **(19),** it may be that the film coating liquid is for coating a tablet.
**(21)** A tablet coated with a film coating liquid according to the present invention may include: a film coating composition according to any one of above **(1)** to **(15);** and a solvent.

### Advantageous Effects

A film coating composition of the present invention can be safe without containing carcinogenic titanium dioxide and can provide an excellent opacity performance at the same level as titanium dioxide, thereby providing a high opacity even with a small amount of coating and can be advantageously used for coating food, formulation, or the like.

In addition, the film coating composition of the present invention and a film coating liquid including the same can have excellent safety and stability per se, and a film coating layer formed by using the same can have excellent adhesiveness, strength, stability, and appearance, thus stably protecting and covering a material to be coated, such as a formulation, food or the like, while having an aesthetically excellent appearance.

### Description of the Drawings

FIG. 1 is a picture of sample tablets (uncoated tablets) and coated tablets prepared in Preparation Example 2.
FIG. 2 is a picture showing the results of evaluating the stability of coated tablets prepared in Preparation Example 2.

### Mode for Invention

Hereinafter, the configuration and effects of the present invention will be described in more detail through Examples. The following Examples are provided only for the purpose of illustrating the present invention, and thus the scope of the present invention is not limited thereto.

### <Examples and Comparative examples>

### Examples 1 to 4 and Comparative example 1. Film coating composition

A film coating composition was prepared in accordance with a composition as shown in table 1 below.

**[Table 1]**

| **Configuration** (wt%) | Comparative Example 1 | Example 1 | Example 2 | Example 3 | Example 4 |
|---|---|---|---|---|---|
| Calcium carbonate (CaCO₃) | 50.0% | 60.0% | 70.0% | 80.0% | 85.0% |
| HPMC 2910 (15 cps) | 43.6% | 34.5% | - | - | - |
| HPMC 2910 (50 cps) | - | - | 24.0% | 14.0% | 9.0% |
| HPMC 2208 (4000 cps) | 4.4% | 3.5% | 3.5% | 3.5% | 4.0% |
| D-sorbitol | 2.0% | 2.0% | 2.5% | 2.5% | 2.0% |

| | | | | | |
|---|---|---|---|---|---|
| Note) Calcium carbonate: Precipitated calcium carbonate HPMC 2910 (15 cps): HPMC 2910 (Pharmacoat 615) with an average viscosity of 15 cps (±20% cps) in an aqueous solution of 2%w/w at 20°C HPMC 2910 (50 cps): HPMC 2910 (Metolose 60SH-50) with an average viscosity of 50 cps (±20% cps) in an aqueous solution of 2%w/w at 20°C HPMC 2208 (4000 cps): HPMC 2208 (Metolose 90SH-4000) with an average viscosity of 4000 cps (±20% cps) in an aqueous solution of 2%w/w at 20°C | | | | | |

### <Preparation Example>

### Preparation Example 1. Preparation of film coating liquid

As a film coating composition, each of Comparative Example 1 and Examples 1 to 4 of above Table 1 was added to purified water, and then dissolved to prepare each of film coating liquid 1 (using Comparative Example 1) and film coating liquids 2 to 5 (using Examples 1 to 4, respectively). It was prepared so that the film coating composition might be included in an amount of 15 parts by weight compared to 100 parts by weight of the total amount of the film coating liquid.

### Preparation Example 2. Preparation of coated tablet

Each of the film coating liquids prepared in above Preparation Example 1 was spray-coated (average spray rate of 3 g/min) on sample tablets by using a coating machine (Labcoat^{™} Benchtop Tablet Coating Systems, O'HARA Technology). After spray coating, the tablets were dried and cooled in the coating machine (at 40°C for 20 minutes) for drying and curing. As a sample tablet (uncoated tablet) to be coated, an iron-containing round tablet (400 mg) was used, and the weight of the tablet after coating was 424 mg.

### <Experimental Example>

An experiment was conducted to confirm the opacity and stability of a coating layer formed by using the film coating liquid prepared by using each of the compositions of above Comparative Examples and Examples.

### Experimental Example 1. Evaluation of opacity and appearance of coating layer

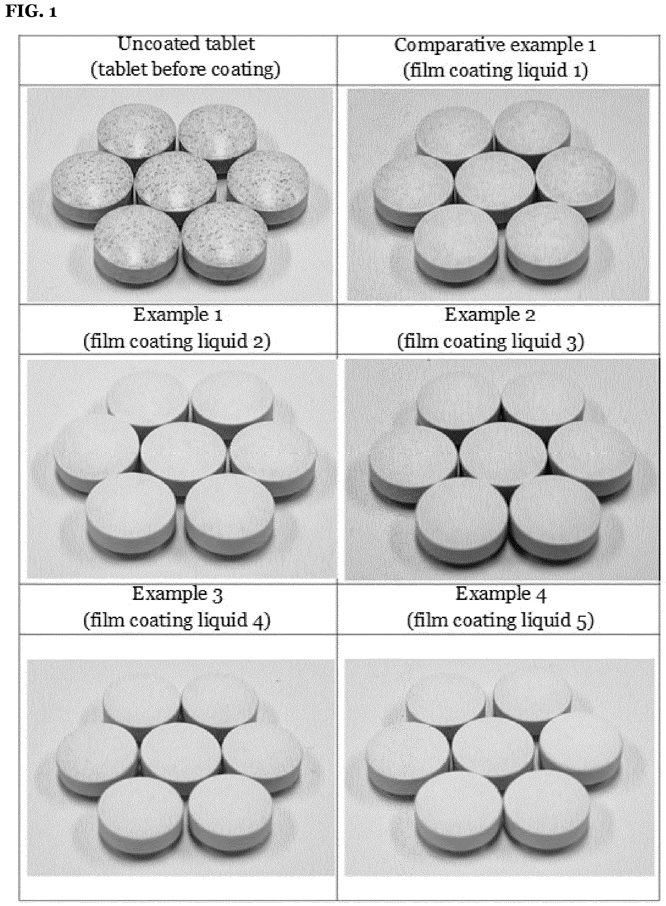

The opacity and appearance of the coating layer formed on each coated tablet prepared in above Preparation Example 2 were evaluated, and the results are shown in FIG. 1 and Table 2.

As can be confirmed from FIG. 1, it was confirmed that the coating layers of the tablets coated with each of film coating liquids 2 to 5 prepared by using each of film coating compositions of Examples 1 to 4 exhibit a high opacity, and thus the surface pattern exhibited in the sample uncoated tablet (tablet before coating) is completely covered, and excellent coating uniformity and whiteness (Table 2) are exhibited.

On the contrary, it was confirmed that the tablets coated with film coating liquid 1 prepared by using the film coating composition of Comparative Example 1 have low whiteness and poor opacity, and thus the pattern of the sample uncoated tablet is not completely covered.

In other words, it was confirmed that the film coating composition of the present invention and the film coating liquid prepared by using the same are safe without containing titanium dioxide and exhibit excellent whiteness and opacity, thereby exhibiting a high opacity even when the same amount of coating is used.

### Experimental Example 2. Stress stability evaluation

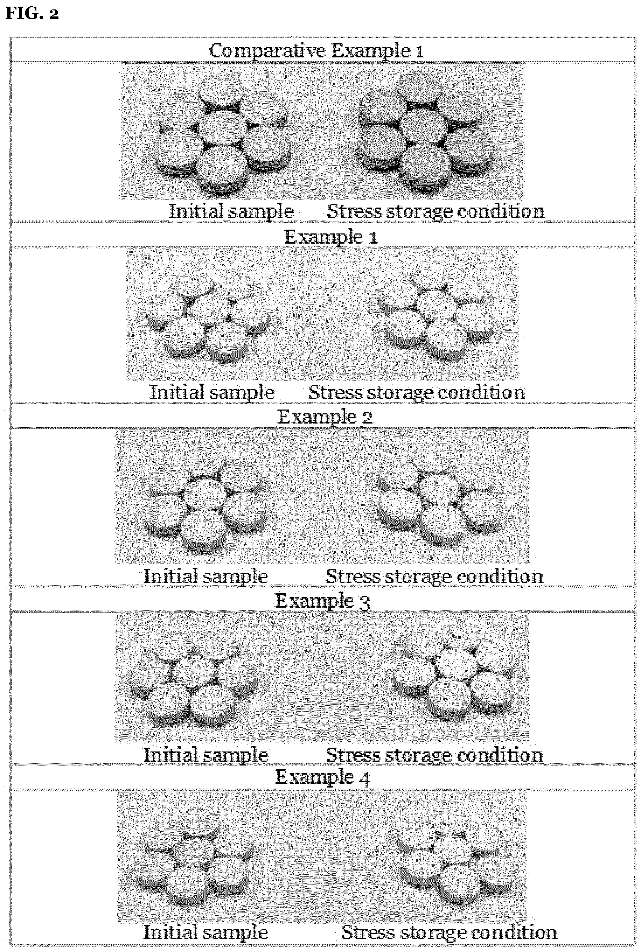

An experiment was conducted to evaluate the stability of the coated tablet under stress conditions. After each coated tablet (initial coated tablet) prepared in above Preparation Example 2 was stored for 168 hours under stress conditions (temperature 40°C, relative humidity 75%), stress stability was evaluated compared to the initial coated tablet, and the results are shown in Table 2 (color coordinates of the coated tablet measured with a CIE LAB color space) and FIG. 2.

**[Table 2]**

| Color coordinates | Initial sample (initial coated tablet) | | | After 168 hours of storage under stress conditions | | | ΔE |
|---|---|---|---|---|---|---|---|
| | L* | a* | b* | L* | a* | b* | |
| Uncoated tablet | 76.6 | -1.6 | 18.8 | - | - | - | - |
| Comparative Example 1 | 87.3 | -0.6 | 9.7 | 82.5 | 0.1 | 5.9 | 6.2 |
| Example 1 | 91.9 | -0.1 | 6.8 | 91.9 | -0.1 | 6.2 | 0.6 |
| Example 2 | 92.8 | 0.0 | 5.7 | 92.1 | 0.1 | 5.0 | 1.0 |
| Example 3 | 93.5 | 0.1 | 4.9 | 93.0 | 0.1 | 4.5 | 0.6 |
| Example 4 | 93.6 | -0.1 | 5.2 | 93.7 | 0.1 | 4.7 | 0.6 |

| | | | | | | | |
|---|---|---|---|---|---|---|---|
| Note) L*: indicates brightness, 0 means black, and 100 means white a*: If the value is negative, the color of the coated tablets deviates towards green. If the value is positive, the color of the coated tablets deviates towards red. b*: If the value is negative, the color of the coated tablets deviates towards blue. If the value is positive, the color of the coated tablets deviates towards yellow. △E: a color difference value obtained by digitizing the color difference between the initial sample and the tablet after storage under stress conditions for 168 hours, and determined that a visually distinguishable change has occurred, if the value is 1.5 or more. | | | | | | | |

As can be confirmed from above Table 2 and FIG. 2, it was found that the coating layer of the tablet coated with the film coating compositions (Examples 1 to 4) of the present invention is maintained without color change even after storage under stress conditions, whereas the tablet coated with the composition of Comparative Example 1 has a visually distinguishable color change after storage under stress conditions. Accordingly, it was confirmed that the film coating composition of the present invention may form a coating layer having excellent stability even under stress conditions.

## Claims

1. A film coating composition comprising:
60 wt% to 85 wt% of calcium carbonate (CaCO₃) and 10 wt% to 38 wt% of hydroxypropyl methylcellulose (HPMC) compared to the total weight of the composition.

2. The film coating composition of claim 1, wherein the hydroxypropyl methylcellulose is a mixture of HPMC having a viscosity of 100 cps or less and HPMC having a viscosity of 3000 cps or more.

3. The film coating composition of claim 1, wherein the hydroxypropyl methylcellulose is a mixture of HPMC having a viscosity of 6 to 100 cps and HPMC having a viscosity of 3000 to 12000 cps.

4. The film coating composition of claim 1, wherein the hydroxypropyl methylcellulose is a mixture of HPMC having a viscosity of 15 to 100 cps and HPMC having a viscosity of 3000 to 10000 cps.

5. The film coating composition of claim 1, wherein the hydroxypropyl methylcellulose is a mixture of HPMC having a viscosity of 15 to 50 cps and HPMC having a viscosity of 3000 to 5000 cps.

6. The film coating composition of claim 2, wherein a weight ratio of the HPMC having a viscosity of 100 cps or less and the HPMC having a viscosity of 3000 cps or more is 1:1 to 15:1.

7. The film coating composition of claim 2, wherein a weight ratio of the HPMC having a viscosity of 100 cps or less and the HPMC having a viscosity of 3000 cps or more is 1:1 to 10:1.

8. The film coating composition of claim 2, wherein the HPMC having a viscosity of 100 cps or less is HPMC 2910.

9. The film coating composition of claim 2, wherein the HPMC having a viscosity of 3000 cps or more is HPMC 2208.

10. The film coating composition of claim 1, wherein the composition comprises a plasticizer.

11. The film coating composition of claim 10, wherein the plasticizer is comprised in an amount of 0.5 wt% to 3 wt% compared to the total weight of the composition.

12. The film coating composition of claim 10, wherein the plasticizer is at least one selected from di-sorbitol, di-mannitol, glycerin, polyethylene glycol, triacetin, acetyltriethyl citrate and triethyl citrate.

13. The film coating composition of claim 1, wherein the composition is used for coating food or formulation.

14. The film coating composition of claim 1, wherein the composition is used for spray coating.

15. A film coating liquid comprising a film coating composition according to any one of claims 1 to 14; and a solvent.

16. The film coating liquid of claim 15, wherein the film coating composition is comprised in an amount of 5 wt% to 25 wt% compared to a total weight of the film coating liquid.

17. The film coating liquid of claim 15, wherein the solvent is water or ethanol.

18. The film coating liquid of claim 15, wherein the film coating liquid is used for coating food or formulation.

19. The film coating liquid of claim 15, wherein the film coating liquid is used for coating tablet.

20. A tablet coated with a film coating liquid comprising a film coating composition according to any one of claims 1 to 14; and a solvent.
